# FASCICULE DE BREVET EUROPEEN

(11) **EP 2 131 906 B1**
(45) Date de publication et mention de la délivrance du brevet: **17.02.2021**
(21) Numéro de dépôt: 08799905.8
(22) Date de dépôt: 26.03.2008
(51) Int. Cl.: A61M 25/00

(54) **SONDE INTRALUMINALE CONDITIONNÉE PRÊTE À L'EMPLOI**
VERPACKTER UND GEBRAUCHSFERTIGER INTRALUMINAL-KATHETER
PACKED AND READY TO USE INTRALUMINAL CATHETER

(30) Priorité: 27.03.2007 FR 0702234
(43) Date de publication de la demande: 16.12.2009
(73) Titulaire: B. Braun Medical SAS, 92100 Boulogne Billancourt (FR)
(72) Inventeur: BEZOU, Pascal, F-28330 Authon Du Perche (FR); COLLIN, Rémi, F-61340 Saint Hilaire Sur Eure (FR)
(74) Mandataire: Ipside
(86) Numéro de dépôt international: PCT/FR2008/050525
(87) Numéro de publication internationale: WO 2008/129222

(56) Documents cités:
- WO-A-99/30761
- WO-A-03/064279
- DE-A1- 10 213 411
- FR-A- 2 896 420
- US-A- 6 059 107
- US-A1- 2005 109 648
- US-A1- 2006 196 783

## Description

La présente invention concerne une sonde intraluminale conditionnée à un état prêt à l'emploi selon la revendication 1.

De telles sondes sont destinées à être introduites dans des canaux corporels, par exemple dans l'urètre.

On utilise des sondes intraluminales sous une forme conditionnée soit prête à l'emploi, soit à mouiller avant utilisation. Comme ce dernier type de sonde a l'inconvénient de compliquer la manipulation par l'utilisateur, on cherche à réaliser des sondes prêtes à l'emploi.

On réalise donc des sondes intraluminales prêtes à l'emploi disposées dans un conditionnement étanche qui contient un fluide de mouillage, provoquant par exemple un gonflement du revêtement hydrophile de la sonde qui a ainsi une glissance élevée. Bien qu'on essaie de retenir le fluide de mouillage du côté du tube de la sonde, par exemple par des tampons absorbants ou par formation de poches de fluide, il arrive que du fluide de mouillage parvienne sur l'embout de la sonde qui doit être tenu à la main, et le rende glissant.

Dans le cas des sondes décrites dans le document EP-935 478, le fluide de mouillage est toujours présent sur l'embout de la sonde qui doit être tenu à la main, et qui est donc glissant.

Un autre inconvénient des systèmes connus est que, lorsque la sonde intraluminale est conditionnée entre deux feuilles de matière plastique soudées, l'ouverture se fait par déchirure du conditionnement à partir d'une amorce de déchirure. Lorsque le conditionnement a ainsi été ouvert, l'embout doit être tenu avec une partie du conditionnement, de sorte que le fluide de mouillage peut passer du côté de l'orifice de l'embout qui peut être mouillé avant d'être saisi.

L'invention a pour objet la solution du problème de la manipulation de ces sondes intraluminales. Plus précisément, l'invention a pour objet d'une part d'éviter l'introduction de tout fluide de mouillage du côté de l'embout opposé au tube de la sonde, et d'autre part de faciliter la manipulation après l'ouverture par déchirure du conditionnement depuis une amorce de déchirure.

Selon l'invention, le problème précité est résolu parce que le conditionnement étanche est fixé autour de l'embout de manière étanche afin que l'embout délimite de part et d'autre deux chambres séparées de manière étanche, et parce que l'embout est fermé par un bouchon.

Plus précisément, l'invention concerne une sonde intraluminale conditionnée à un état prêt à l'emploi, du type qui comprend un tube portant un revêtement hydrophile et ayant une extrémité distale d'insertion et une extrémité proximale de maintien raccordée à un embout, celui-ci ayant un canal qui communique d'un côté avec le tube et qui débouche de l'autre côté par un orifice, la sonde étant entièrement disposée dans un conditionnement étanche ; selon l'invention, la sonde conditionnée comprend un bouchon amovible qui ferme de façon étanche l'orifice de l'embout, le conditionnement est fixé de façon étanche à l'embout afin que celui-ci délimite, dans le conditionnement, deux chambres étanches qui ne communiquent que par la lumière du tube et le canal de l'embout, une première chambre dans laquelle est disposé le tube contient un fluide de mouillage du revêtement hydrophile du tube pour lui donner une glissance élevée, et la seconde chambre dans laquelle débouche l'orifice ne contient pratiquement pas de fluide de mouillage.

De préférence, le conditionnement est formé de deux feuilles de matière plastique fixées de manière étanche autour de l'embout.

De préférence, la fixation est effectuée par soudage ou collage.

De préférence, les deux feuilles de matière plastique du conditionnement ont au moins une amorce de déchirure transversale de chaque côté de l'embout.

De préférence, les deux feuilles de matière plastique du conditionnement ont deux amorces de déchirure du côté du tube, l'une proche de l'embout et l'autre plus éloignée.

De préférence, les feuilles de conditionnement ont une faible perméabilité à la vapeur d'eau.

De préférence, les feuilles de conditionnement sont formées d'un polymère orienté dont la direction d'étirage est transversale à la direction du tube.

De préférence, le fluide de mouillage est une solution aqueuse.

De préférence, le revêtement hydrophile du tube gonfle en présence d'eau.

Ainsi, un avantage de l'invention est que le côté de la sonde opposé au tube (embout) est toujours protégé contre la présence du fluide de mouillage, si bien que le maintien commode de la sonde et son raccordement à un ensemble récepteur sont toujours faciles.

D'autres caractéristiques et avantages de l'invention seront mieux compris à la lecture de la description qui va suivre d'exemples de réalisation, faite en référence au dessin annexé sur lequel :
la figure 1 est une vue en plan d'un exemple de sonde intraluminale conditionnée selon l'invention ;
la figure 2 est une vue en perspective d'un exemple d'embout selon l'invention ; et
la figure 3 est une vue en perspective d'une variante d'embout.

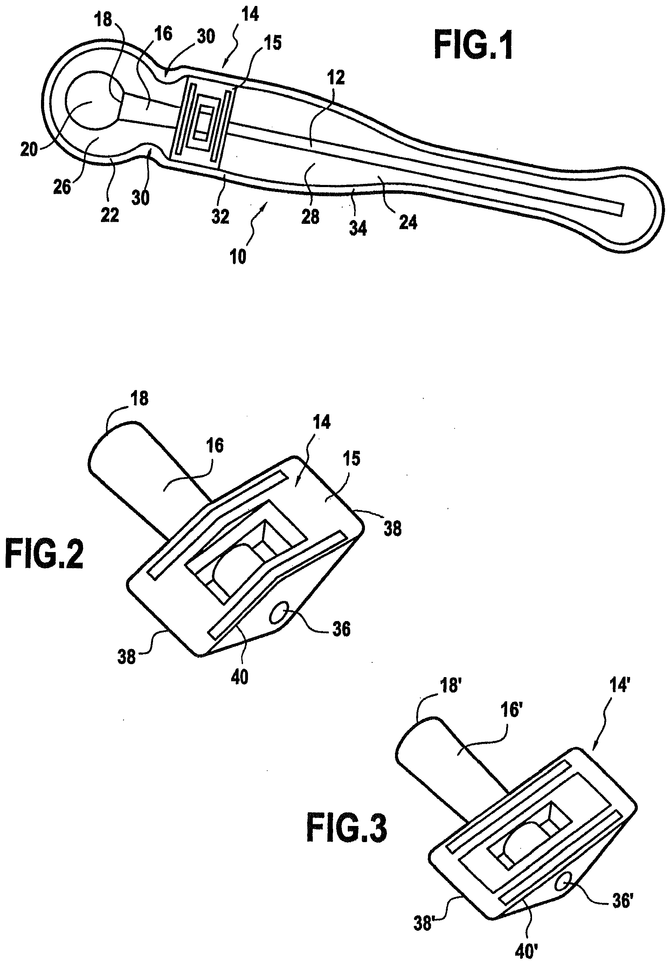

La figure 1 représente une sonde intraluminale conditionnée selon l'invention. Cette sonde 10 comporte un tube 12, portant à sa surface extérieure un revêtement hydrophile de glissance élevée lorsque le revêtement est mouillé, un embout 14 constitué d'une embase 15 et d'un tronçon 16 de conduit qui débouche par un orifice 18 du côté opposé au tube 12.

Il faut noter sur la figure 2 que l'orifice 18 constitue le débouché d'un canal qui passe dans l'embout 14 jusqu'à un orifice 36 communiquant avec la lumière du tube 12 de la sonde.

Sur la figure 1, la référence 20 désigne un bouchon amovible placé sur l'orifice 18 et fermant celui-ci de manière étanche.

Selon l'invention, les deux feuilles du conditionnement 22 forment deux chambres séparées de manière étanche, une première chambre 24 dans laquelle est disposé le tube 12 et une seconde chambre 26 dans laquelle sont disposés le tronçon 16 et le bouchon 20. L'étanchéité entre les deux chambres est obtenue par soudage ou collage des deux feuilles au corps de l'embout 14 et l'une sur l'autre.

Comme l'indique la figure 2, le corps de l'embout 14 a une section en losange se terminant à deux bords effilés 38 et formant, entre les deux bords 38, une surface continue 40 qui peut être soudée ou collée à une feuille du conditionnement. Dans le cas d'un soudage, la feuille de matière plastique transparente du conditionnement et la matière plastique de l'embout 14 sont de préférence de même nature chimique, par exemple de polyéthylène.

Le conditionnement de la sonde intraluminale selon l'invention contient, dans la chambre 24 dans laquelle se trouve le tube 12, un fluide de mouillage permettant au tube 12 de posséder une glissance élevée. Ce fluide est avantageusement une solution aqueuse, de type bien connu dans la technique, dont la composition dépend de la nature exacte du revêtement hydrophile du tube 12. Ainsi, bien que les feuilles du conditionnement soient fixées de manière étanche à l'embout 14, les deux chambres 24, 26 communiquent par l'intérieur du tube 12 et le canal de l'embout 14, jusqu'à l'orifice 18. Pour que le fluide contenu dans la chambre 24 ne puisse pas parvenir à la chambre 26, un bouchon 20 est placé sur l'orifice 18 qu'il ferme de manière étanche.

Sur la figure 1, les références 30, 32 et 34 désignent des amorces de déchirure permettant la rupture du conditionnement en direction transversale à la direction du tube 12.

La figure 3 représente une variante d'embout, ayant un côté plan parfois utile pour la manipulation. Les références suivies du signe ' désignent des éléments analogues à ceux de la figure 2, dans la variante de la figure 3. On ne décrit donc pas plus en détail cette variante.

On note sur la figure 1 que, lorsque la sonde doit être utilisée, elle peut être dégagée par enlèvement de la partie du conditionnement qui se trouve entre l'amorce de déchirure 32 et l'extrémité voisine de l'extrémité d'insertion du tube 12.

Cependant, selon l'invention, il est avantageux de séparer le conditionnement d'abord au niveau de l'amorce 34, si bien que l'extrémité d'insertion du tube est dégagée. Ensuite, la partie restante du conditionnement est déchirée à partir de l'amorce de déchirure 32. Le tronçon du conditionnement compris entre les amorces 32 et 34 constitue alors une sorte de manchon qui peut être pincé entre deux doigts pour faciliter la manipulation de la sonde. Ainsi, l'utilisateur tient normalement l'embase 15 de l'embout 14 de la main gauche et le manchon compris entre les amorces 32 et 34 de la main droite. En déplaçant ce manchon vers l'extrémité d'insertion, l'utilisateur tient avec une bonne précision l'extrémité du tube à insérer, et ce sans le toucher avec les doigts (technique "no touch"). Au fur et à mesure que cette insertion se poursuit, par application d'une force le long du tube, la main droite guide la gaine vers l'embase jusqu'à la profondeur d'insertion convenable.

A ce moment, ou avant l'opération précédente, la partie de conditionnement placée de l'autre côté de l'embout 14 peut être déchirée au niveau de l'amorce 30. L'utilisateur, qui tient toujours l'embase de la main gauche, peut alors retirer le bouchon 20 et relier l'embout à un dispositif destiné à recevoir le fluide évacué par la sonde.

Il est avantageux de retirer la partie de conditionnement placée de l'autre côté de l'embout 14 et le bouchon en une seule opération.

Dans cette dernière opération, le fluide de mouillage placé autour du tube 12 est retenu par la partie de conditionnement comprise entre l'embase et l'amorce 32, si bien que le fluide ne risque pas de venir souiller le tronçon de tube 16 ni le bouchon 20, et la manipulation est très sûre et commode.

La fabrication des sondes selon l'invention ne présente pas de difficultés techniques, car il est simple de souder les feuilles de conditionnement sur l'embout pendant l'opération de soudage des feuilles l'une sur l'autre, lors de la fermeture étanche du conditionnement. Dans un mode de réalisation, cette opération de soudage est réalisée d'abord par soudage des deux feuilles sur l'embout, puis par soudage des bords des feuilles, sauf de l'extrémité la plus proche de l'extrémité d'insertion du tube 12. En effet, une fois le conditionnement fermé sauf à cette extrémité, le fluide de mouillage destiné à conserver les propriétés de glissance du revêtement hydrophile du tube 12 est introduit, et l'extrémité est seulement soudée à ce moment.

La stérilisation peut être effectuée par les procédés classiques, sans modification.

Bien qu'on ait décrit un conditionnement formé de deux feuilles soudées, il est évident qu'il peut être formé d'une seule feuille repliée et soudée sur elle-même.

## Revendications

1. Sonde intraluminale conditionnée à un état prêt à l'emploi, du type qui comprend un tube (12) portant un revêtement hydrophile et ayant une extrémité distale d'insertion et une extrémité proximale de maintien raccordée à un embout (14), celui-ci ayant un canal qui communique d'un côté avec le tube (12) et qui débouche de l'autre côté par un orifice (18), la sonde étant entièrement disposée dans un conditionnement étanche, **caractérisée en ce que** :
- le conditionnement est fixé de façon étanche à l'embout (14) afin que celui-ci délimite, dans le conditionnement, une première chambre (24) et une seconde chambre (26), séparées de manière étanche et qui ne communiquent que par la lumière du tube (12) et le canal de l'embout (14),
- la première chambre (24), dans laquelle est disposé le tube, contient un fluide de mouillage du revêtement hydrophile du tube (12) pour lui donner une glissance élevée, et
- la seconde chambre (26) dans laquelle débouche l'orifice (18) comprend un bouchon amovible (20) qui ferme de façon étanche l'orifice (18) de l'embout (14) pour que le fluide contenu dans la chambre première chambre (24) ne puisse pas parvenir à la seconde chambre (26).

2. Sonde intraluminale selon la revendication 1, **caractérisée en ce que** le conditionnement est formé de deux feuilles de matière plastique fixées de manière étanche autour de l'embout (14).

3. Sonde intraluminale selon la revendication 2, **caractérisée en ce que** la fixation est effectuée par soudage ou collage.

4. Sonde intraluminale selon l'une quelconque des revendications 1 à 3, **caractérisée en ce que** les deux feuilles de matière plastique du conditionnement ont au moins une amorce (30, 32, 34) de déchirure transversale de chaque côté de l'embout.

5. Sonde intraluminale selon la revendication 4, **caractérisée en ce que** les deux feuilles de matière plastique du conditionnement ont deux amorces (32, 34) de déchirure du côté du tube (12), l'une (30) proche de l'embout et l'autre (34) plus éloignée.

6. Sonde intraluminale selon l'une quelconque des revendications 2 à 5, **caractérisée en ce que** les feuilles de conditionnement ont une faible perméabilité à la vapeur d'eau.

7. Sonde intraluminale selon l'une quelconque des revendications précédentes, **caractérisée en ce que** le fluide de mouillage est une solution aqueuse.

8. Sonde intraluminale selon l'une quelconque des revendications précédentes, **caractérisée en ce que** le revêtement hydrophile du tube (12) est sélectionné parmi les revêtements qui possèdent des propriétés de gonflement en présence d'eau.

## Patentansprüche

1. In einem gebrauchsfertigen Zustand verpackte intraluminale Sonde des Typs, der eine Röhre (12) umfasst, die eine hydrophile Beschichtung trägt und ein distales Einführende und ein proximales Halteende aufweist, das mit einem Ansatz (14) verbunden ist, wobei dieser einen Kanal aufweist, der auf einer Seite mit der Röhre (12) kommuniziert und der auf der anderen Seite durch eine Öffnung (18) ausmündet, wobei die Sonde vollständig in einer dichten Verpackung angeordnet ist, **dadurch gekennzeichnet, dass**:
- die Verpackung dicht am Ansatz (14) befestigt ist, sodass dieser in der Verpackung eine erste Kammer (24) und eine zweite Kammer (26) abgrenzt, die dicht abgetrennt sind und die nur durch das Lumen der Röhre (12) und den Kanal des Ansatzes (14) kommunizieren,
- die erste Kammer (24), in der die Röhre angeordnet ist, ein Fluid zum Benetzen der hydrophilen Beschichtung der Röhre (12) enthält, um dieser eine hohe Gleitfähigkeit zu verleihen, und
- die zweite Kammer (26), in die die Öffnung (18) ausmündet, einen abnehmbaren Stopfen (20) umfasst, der die Öffnung (18) des Ansatzes (14) dicht verschließt, damit das in der ersten Kammer (24) enthaltene Fluid nicht in die zweite Kammer (26) gelangen kann.

2. Intraluminale Sonde nach Anspruch 1, **dadurch gekennzeichnet, dass** die Verpackung aus zwei Kunststofffolien gebildet ist, die dicht um den Ansatz (14) herum befestigt sind.

3. Intraluminale Sonde nach Anspruch 2, **dadurch gekennzeichnet, dass** die Befestigung durch Schweißen oder Kleben erfolgt.

4. Intraluminale Sonde nach einem der Ansprüche 1 bis 3, **dadurch gekennzeichnet, dass** die zwei Kunststofffolien der Verpackung auf jeder Seite des Ansatzes mindestens eine quer verlaufenden Aufreißkerbe (30, 32, 34) aufweisen.

5. Intraluminale Sonde nach Anspruch 4, **dadurch gekennzeichnet, dass** die zwei Kunststofffolien der Verpackung auf der Seite der Röhre (12) zwei Aufreißkerben (32, 34) aufweisen, eine (30) nahe am Ansatz und die andere (34) weiter entfernt.

6. Intraluminale Sonde nach einem der Ansprüche 2 bis 5, **dadurch gekennzeichnet, dass** die Verpackungsfolien eine geringe Wasserdampfdurchlässigkeit aufweisen.

7. Intraluminale Sonde nach einem der vorstehenden Ansprüche, **dadurch gekennzeichnet, dass** es sich beim Benetzungsfluid um eine wässrige Lösung handelt.

8. Intraluminale Sonde nach einem der vorstehenden Ansprüche, **dadurch gekennzeichnet, dass** die hydrophile Beschichtung der Röhre (12) aus den Beschichtungen ausgewählt ist, die in Gegenwart von Wasser quellende Eigenschaften aufweisen.

## Claims

1. An intraluminal probe packaged in a ready-to-use state, of the type comprising a tube (12) bearing a hydrophilic coating and having a distal insertion end and a proximal holding end connected to a tip (14), the tip having a channel which communicates on one side with the tube (12) and which opens on the other side via an orifice (18), the probe being entirely placed in a sealed packaging, **characterised in that**:
- the packaging is attached in a sealed manner to the tip (14) so that it defines, in the packaging, a first chamber (24) and a second chamber (26), sealingly separated and which communicate only through the lumen of the tube (12) and the channel of the tip (14),
- the first chamber (24), in which the tube is placed, contains a fluid for wetting the hydrophilic coating of the tube (12) which gives it high slipperiness,
and
- the second chamber (26) into which the orifice (18) opens comprises a removable plug (20) which sealingly closes the orifice (18) of the tip (14) so that the fluid contained in the first chamber (24) cannot reach the second chamber (26).

2. Intraluminal probe according to claim 1, **characterised in that** the packaging is formed of two sheets of plastic material sealingly attached around the tip (14).

3. Intraluminal probe according to claim 2, **characterised in that** the attachment is made by welding or bonding.

4. Intraluminal probe according to any one of claims 1 to 3, **characterised in that** the two sheets of plastic material of the packaging have at least one transverse tear tab (30, 32, 34) on each side of the tip.

5. Intraluminal probe according to claim 4, **characterised in that** the two sheets of plastic material of the packaging have two tear tabs (32, 34) on the side of the tube (12), one (30) close to the tip and the other (34) further away.

6. Intraluminal probe according to any of claims 2 to 5, **characterised in that** the packaging sheets have a low permeability to water vapour.

7. Intraluminal probe according to any one of the preceding claims, **characterised in that** the wetting fluid is an aqueous solution.

8. Intraluminal probe according to any one of the preceding claims, **characterised in that** the hydrophilic coating of the tube (12) is selected among the coatings which have swelling properties in the presence of water.
